# EUROPEAN PATENT SPECIFICATION

(11) **EP 3 476 391 B1**
(45) Date of publication and mention of the grant of the patent: **11.08.2021**
(21) Application number: 17815505.7
(22) Date of filing: 23.06.2017
(51) Int. Cl.: A61K 31/198, A61P 21/02, A61P 25/00, A61P 25/14, A61P 21/00, A61P 25/28

(54) **PHARMACEUTICAL COMPOSITION COMPRISING ARGININE FOR USE IN TREATING POLYQ DISEASE**
PHARMAZEUTISCHE ZUSAMMENSETZUNG ENTHALTEND ARGININ ZUR VERWENDUNG BEI DER BEHANDLUNG DER POLYQ-KRANKHEIT
COMPOSITION PHARMACEUTIQUE COMPRENANT DE L'ARGININE POUR L'UTILISATION DANS LE TRAITEMENT DE LA MALADIE DE POLYQ

(30) Priority: 23.06.2016 JP 2016124992
(43) Date of publication of application: 01.05.2019
(73) Proprietor: Japan Science and Technology Agency, Kawaguchi-shi, Saitama 332-0012 (JP)
(72) Inventor: NAGAI Yoshitaka, Suita-shi Osaka 565-0871 (JP); POPIEL Helena Akiko, Tokyo 160-0023 (JP); MINAKAWA Eiko, Kodaira-shi Tokyo 187-8551 (JP)
(74) Representative: Plasseraud IP
(86) International application number: PCT/JP2017/023162
(87) International publication number: WO 2017/222040

(56) References cited:
- WO-A1-2015/061856
- US-A1- 2004 152 778
- DECKEL A. W. et al.: "Dietary arginine alters time of symptom onset in Huntington's desease transgenic mice", Brain Research, vol. 875, 2000, pages 187-195, XP055446847,
- NAKAYAMA IZUMI et al.: "Inhibition of polyglutamine aggregation by arginine and its effect on a polyglutamine disease mouse", Bulletin of the Japanese Society for Neurochemistry, vol. 45, no. 2-3, 25 August 2006 (2006-08-25), page 455, XP009514221,

## Description

### Technical Field

The present invention relates to a pharmaceutical composition as defined in the appended claims effective as a therapeutic agent for use in treatment or prevention of the polyglutamine (PolyQ) diseases as defined in the appended claims, which are neurodegenerative diseases selected from the group consisting of various inherited spinocerebellar ataxias (SCAs), spinal bulbar muscular atrophy (SBMA)and dentatorubral palliodoluysian atrophy, and can be safely administered to humans.

### Background Art

Along with the aging of the population, neurodegenerative diseases that develop with aging have been increasing steadily and have become a serious social problem because there is no effective treatment. For this reason, it is required that effective pharmaceutical agents for these neurodegenerative diseases are developed.

The PolyQ diseases are one group of neurodegenerative diseases that develop with aging. The PolyQ diseases are a generic term for nine hereditary neurodegenerative diseases, including Huntington disease, and various spinocerebellar ataxias, which are caused by an abnormal expansion (> 40 amino acids) of PolyQ stretch in the disease-causing proteins. The PolyQ diseases are intractable diseases that still have no effective treatment, although it is estimated that about 7,000 to 8,000 people are affected in Japan. In the polyQ diseases, the expansion of polyQ stretch within the disease-causing proteins triggers misfolding and aggregation of these proteins, resulting in neurodegeneration.

Chemical chaperones stabilize proteins in their natural conformation and exert anti-aggregating properties by affecting the rate or fidelity of the protein folding reaction. Arginine is a chemical chaperone and is one of the most commonly used additives to prevent the aggregation and increase the solubility of various recombinant proteins expressed in Escherichia coli (Non-Patent Document 1). This nonspecific anti-aggregation effect of arginine indicates the possibility that arginine could be utilized for the treatment of protein misfolding diseases in general including Alzheimer disease and Parkinson disease. Indeed, it is reported that arginine prevents aggregation of amyloid-β (beta) 1-42 (Non-Patent Document 2).

Arginine is one of the amino acids constituting organisms including humans, and can be safely administered to animals such as humans. The safety of arginine has been established in clinical trials on diseases other than the PolyQ diseases (Non-Patent Documents 3 and 4). Furthermore, arginine is known to cross the blood brain barrier (BBB) (Non-Patent Document 5).

### Citation List

### Non-Patent Literature

[Non-Patent Document 1] Arakawa et al. , Biochemical and Biophysical Research Communications, 2003, vol.304(1), p.148-152.
[Non-Patent Document 2] Das et al., PLoS One, November 2007, Issue 11, e1176.
[Non-Patent Document 3] Koga et al., Neurology, 2002, vol.58(5), p.827.
[Non-Patent Document 4] Boenzi et al., Journal of Inherited Metabolic Disease, 2012, vol.35(4), p.647-653.
[Non-Patent Document 5] Pardridge, Physiological Reviews, 1983, vol.63(4), p.1481-1535.
[Non-Patent Document 6] Nagai et al., JOURNAL OF BIOLOGICAL CHEMISTRY, 2000, vol.275(14), p.10437-10442.
[Non-Patent Document 7] Nagai et al., Nature Structural & Molecular Biology, 2007, vol.14(4), p.332-340.
[Non-Patent Document 8] Jarrett and Lansbury, Cell, 1993, vol.73(6), p.1055-1058.
[Non-Patent Document 9]Williams and Paulson, Trends in Neurosciences, 2008, vol.31(10), p.521-528.
[Non-Patent Document 10] Takahashi et al., Human Molecular Genetics, 2008, vol.17(3), p.345-356.
[Non-Patent Document 11] Takahashi et al., JOURNAL OF BIOLOGICAL CHEMISTRY, 2007, vol.282(33), p.24039-24048.
[Non-Patent Document 12] Warrick et al., Cell, 1998, vol.93(6), p.939-949.
[Non-Patent Document 13] Nagai et al., Human Molecular Genetics, 2003, vol.12(11), p.1253-1260.
[Non-Patent Document 14] Satyal et al., Proceedings of the National Academy of Sciences of the United States of America, 2000, vol.97(11), p.5750-5755.
[Non-Patent Document 15] Watase et al., Neuron, 2002, vol.34(6), p.905-919.
[Non-Patent Document 16] Hamase et al., Journal of Chromatography A, 2010, vol.1217(7), p.1056-1 062.
[Non-Patent Document 17] Popiel et al., PLoS One, 2012, vol.7(11), e51069.
[Non-Patent Document 18] Katsuno et al., Neuron, 2002, vol.35(5), p.843-854.
[Non-Patent Document 19] Katsuno et al., Nature Medicine, 2003, vol.9(6), p.768-773.
[Non-Patent Document 20] Deckel et al. (Brain Research, 2000, 875, 187-195) specifically relates to the treatment of Huntington disease.
[Patent Document 21] US 2004/152778 discloses the treatment of polyglutamine disorders by the administration of effective amounts of L-methionine S-sulfoximine, L-ethionine S-sulfoximine, glufosinate and/or branched chain alpha-keto acids.
[Non-Patent Document 22] Nakayama Izumi et al. (Bulletin of the Japanese Society for Neurochemistry, 2006, 45, 455-) discloses that arginine inhibits polyQ aggregation in vitro but oral administration of arginine in a mouse model of Huntington disease showed no improvement in motor neuron impairment and body weight loss.

### Summary of Invention

### Technical Problem

The objective of the present invention is to provide a pharmaceutical composition safely administrable to humans and effective for treatment of the PolyQ diseases selected from the group consisting of inherited spinocerebellar ataxias, dentatorubral palliodoluysian atrophy, and spinal bulbar muscular atrophy.

### Solution to Problem

The inventors of the present invention have screened PolyQ aggregation inhibiting compounds using an in vitro assay system to develop a common therapeutic agent targeting misfolding and aggregation of denatured proteins, and have identified arginine as a PolyQ aggregation inhibiting compound with high BBB permeability and safety. Furthermore, the inventors have found that arginine suppresses PolyQ aggregate formation and neurodegeneration not only in cultured cell models but also in Drosophila and mouse models of spinocerebellar ataxias (SCA1, SCA3) and spinal bulbar muscular atrophy (SBMA). Based on the findings, the present invention was completed.

The pharmaceutical composition for use according to the present invention is the following [1] to [2].
[1] A pharmaceutical composition including: one selected from the group consisting of arginine, a physiologically acceptable salt thereof, and a solvate thereof, as an active ingredient, for use in the treatment or prevention of a PolyQ disease, in which the PolyQ disease is one selected from the group consisting of inherited spinocerebellar ataxias, dentatorubral palliodoluysian atrophy, and spinal bulbar muscular atrophy.
[2] The pharmaceutical composition for use according to [1], in which the PolyQ disease is one selected from the group consisting of inherited spinocerebellar ataxia type 1, inherited spinocerebellar ataxia type 3, and spinal bulbar muscular atrophy.

### Advantageous Effects of Invention

Arginine is a physiologically active substance that has been confirmed to be safe in humans and has high BBB permeability. For this reason, the pharmaceutical composition for use according to the present invention containing arginine as an active ingredient is very effective for treating the PolyQ diseases selected from the group consisting of inherited spinocerebellar ataxias, dentatorubral palliodoluysian atrophy, and spinal bulbar muscular atrophy in various animals including humans.

### Brief Description of Drawings

FIG. 1 is a graph showing the measurement results of turbidity over time when the Thio-Q62 protein was incubated for 5 days in the presence (closed circles) or absence (open circles) of arginine in Example 1.
FIG. 2 is a graph showing the relative turbidity (%) of each solution after incubating the Thio-Q62 protein in the presence of arginine or proline for 5 days (turbidity of the solution containing only the Thio-Q62 protein is taken as 100%) in Example 1.
FIG. 3 is a graph showing the measurement results of the turbidity over time of a solution containing only the Thio-Q62 protein, a solution containing the Thio-Q62 protein with addition of seed alone, a solution containing the Thio-Q62 protein with addition of seed and arginine, and a solution containing the Thio-Q62 protein with addition of seed and proline in Example 1.
FIG. 4 is a circular dichroism (CD) spectrum of each solution containing the Thio-Q62 protein alone before and after incubation for 5 days in Example 2.
FIG. 5 is an image of CBB-stained native PAGE gel of the Thio-Q62 protein incubated for 5 days in the presence or absence of arginine in Example 2.
FIG. 6 is a graph showing the results of measuring the ratio of fluorescence resonance energy transfer (FRET) positive cells among cells simultaneously expressing Q56-CFP and Q56-YFP incubated with or without arginine in Example 2.
FIG. 7 is a graph showing the measurement results of counts per particle (CPP) obtained by fluorescence correlation spectroscopy (FCS) analysis of cell lysates of cells expressing Q45-GFP in Example 2.
FIG. 8 is a graph showing the measurement results of diffusion time of the fast components (DT₁) obtained by FCS analysis of cell lysates of cells expressing Q45-GFP in Example 2.
FIG. 9 is representative light microscopic images of the external compound eyes of the wildtype and MJDtr-Q78(S) flies in Example 3.
FIG. 10 is immunostaining images with an anti-hemagglutinin antibody of the eye discs of larvae of MJDtr-Q78(S) flies in Example 3.
FIG. 11 is a graph showing the measurement results of tail movement per minute (count / min) obtained from the tail flick test on the PolyQ disease model line of C. elegans in Example 3.
FIG. 12 is a graph showing the time course of the survival rate of the PolyQ disease model line of C. elegans in Example 3.
FIG. 13 is an SDD-AGE image obtained by immunoblotting of the nematode lysates in Example 3.
FIG. 14 is a graph showing the measurement results of smear intensity in the SDD-AGE image of FIG. 13.
FIG. 15 is a graph showing the measurement results of the amount of L-arginine in the cerebrum of SCA1 mice in Example 4.
FIG. 16 is a graph showing the measurement results of the amount of L-arginine in the serum of SCA1 mice in Example 4.
FIG. 17 is a graph showing the time course of the walking time until wild type mice and SCA1 mice fell from the rotarod (Latency) in the rotarod test of Example 4.
FIG. 18 is a graph showing the time course of the daily water intake of each mouse in the rotarod test of Example 4.
FIG. 19 is a graph showing the time course of the amount of time to cross the beam of 28-week-old wild type mice and SCA1 mice in the balance beam test of Example 4.
FIG. 20 is a graph showing the results of measuring the percentage of cells with inclusion body formation in the cerebral cortex and hippocampus of SCA1 mice by immunohistochemical analyses of Example 4.
FIG. 21 is a graph showing the time course of the walking time until the wild type mice and SCA1 mice fell from the rotarod (Latency) in the rotarod test of Example 5.
FIG. 22 is a graph showing the observation results of horizontal activity of wild-type mice and SBMA mice in Example 5.
FIG. 23 is a graph showing the observation results of rearing behavior of wild-type mice and SBMA mice in Example 5.

### Description of Embodiments

The pharmaceutical composition according to the present invention is for use in the treatment or prevention of the PolyQ diseases selected from the group consisting of inherited spinocerebellar ataxias, dentatorubral palliodoluysian atrophy, and spinal bulbar muscular atrophy, and contains arginine as the active ingredient. Arginine has aggregation inhibitory effect of the disease-causing proteins with an abnormally expanded PolyQ stretch. This effect suppresses PolyQ aggregate formation and neurodegeneration. In addition, arginine has been confirmed to be safe in humans and has high BBB permeability. Therefore, the pharmaceutical composition for use according to the present invention can safely and efficiently inhibit PolyQ aggregate-formation and the like in the central nervous system.

The active ingredient contained in the pharmaceutical composition for use according to the present invention may be a physiologically acceptable salt of arginine. Arginine is a basic amino acid and the physiologically acceptable salts of arginine include, for example, salts of mineral acids such as hydrochloric acid, hydrobromic acid, sulfuric acid, nitric acid, and phosphoric acid; salts of organic acids such as methanesulfonic acid, benzenesulfonic acid, p-toluenesulfonic acid, acetic acid, propionate, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts of acidic amino acids such as aspartic acid and glutamic acid.

The active ingredient contained in the pharmaceutical composition for use according to the present invention may be a solvate of arginine or a physiologically acceptable salt thereof. Examples of the solvent that forms the solvate include water, ethanol and the like.

The active ingredient contained in the pharmaceutical composition for use according to the present invention may be only free form of arginine. The active ingredient may be both a free form of arginine and its physiologically acceptable salt. The active ingredient may be both a free form of arginine and its solvate. The active ingredient may be both a physiologically acceptable salt of arginine and its solvate. The active ingredient may be all of a free form of arginine, a physiologically acceptable salt thereof and their solvates.

The pharmaceutical composition for use according to the present invention may comprise only the active ingredient, which is arginine, its physiologically acceptable salt, or their solvates. In addition to the active ingredient, the pharmaceutical composition for use according to the present invention may contain various additives as long as they do not impair the PolyQ aggregation inhibitory effect of arginine. Examples of such additives include excipients, binders, lubricants, wetting agents, solvents, disintegrating agents, solubilizing agents, suspending agents, emulsifying agents, isotonizing agents, stabilizers, buffer, preservative, antioxidant, flavoring, and coloring agent. These additives can be appropriately selected from pharmaceutically acceptable substances which are used for pharmaceutical formulation.

The pharmaceutical composition for use according to the present invention may contain other active ingredients as long as it does not impair the PolyQ aggregation inhibitory effect of arginine. Examples of such other active ingredients include other active ingredients of therapeutic agents for the PolyQ diseases.

The pharmaceutical composition for use according to the present invention is extremely effective as a component of medicine for treatment against the PolyQ diseases selected from the group consisting of inherited spinocerebellar ataxias, dentatorubral palliodoluysian atrophy, and spinal bulbar muscular atrophy, ie, diseases caused by aggregation of proteins with an abnormally expanded PolyQ stretch.

Currently, inherited spinocerebellar ataxias (SCA1, SCA2, SCA3, SCA6, SCA7, SCA17), dentatorubral palliodoluysian atrophy (DRPLA), and spinal bulbar muscular atrophy (SBMA), are eight known neurodegenerative diseases. The pharmaceutical composition for use according to the present invention is effective as a therapeutic agent for any of these eight diseases, and is particularly useful as a component of medicine for treatment or prevention of SCA1, SCA3, or SBMA.

Spinocerebellar ataxias is the generic name for sporadic or inherited neurodegenerative diseases in which the main symptom is ataxia. Clinically, its main symptom is cerebellar or posterior ataxia or spastic paraparesis. In spinocerebellar degeneration, cerebellum and brainstem atrophy are often seen and features such as basal ganglia lesions and cerebral cortical atrophy may be observed.. SBMA is an inherited disease due to the expansion of CAG repeats encoding the PolyQ stretch in the androgen receptor gene. In SBMA, neurological findings such as bulbar symptoms, lower motor neuron signs, tremor and limb tendon reflex reduction are observed, and androgen deficiency symptoms and neurogenic changes may be observed.

Arginine is a chemical chaperone that acts on protein folding. Therefore, the pharmaceutical composition disclosed herein is effective not only against the PolyQ diseases but is also effective as a component of medicine for treatment or prevention against neurodegenerative diseases caused by abnormal protein aggregation. Examples of such neurodegenerative diseases include Alzheimer's disease in which aggregation of amyloid-β or tau occurs, Parkinson's disease in which aggregation of α (alfa)-synuclein occurs, amyotrophic lateral sclerosis in which aggregation of TDP-43 (TAR DNA-binding protein of 43 kD) occurs, and frontotemporal lobar degeneration in which aggregation of TDP-43 or tau occurs. Indeed, arginine has an inhibitory effect on aggregation of amyloid-β, which is a disease-causing protein of Alzheimer's disease.

The pharmaceutical composition for use according to the present invention is preferably administered to humans or non-human animals. Examples of the non-human animals include mammals such as cattle, swine, horse, sheep, goat, monkey, dog, cat, rabbit, mouse, rat, hamster, and guinea pig; birds such as chickens, quail and ducks. The pharmaceutical composition for use according to the present invention may also be administered to invertebrates such as flies and nematodes, and may be administered to cultured cells, yeast, filamentous fungi and the like.

The method for administering the pharmaceutical composition for use according to the present invention to an animal is not particularly limited, and the dosage of thereof is also not particularly limited. Examples of dosage forms suitable for oral administration include capsules, powders, tablets, granules, fine granules, emulsions, syrups, solutions, and suspensions. Examples of dosage forms suitable for parenteral administration include inhalants, sprays, intrarectal agents, injections, drip infusions, ointments, creams, transdermal absorbents, transmucosal absorbents, eye drops, nose drops, ear drops, tape agents, and patches.

Among the pharmaceutical compositions suitable for oral administration, solid preparations such as capsules, tablets, powders, and granules may be produced using additives for formulation such as excipients such as lactose, glucose, sucrose, and mannitol; disintegrating agents such as starch and sodium alginate; lubricants such as magnesium stearate and talc; binders such as polyvinyl alcohol, hydroxypropyl cellulose, and gelatin; surfactants such as fatty acid ester; plasticizers such as glycerin. Liquid preparations such as emulsions and syrups may be produced using additives for formulation such as water; saccharides such as sucrose, sorbitol and fructose; glycols such as polyethylene glycol and propylene glycol; oils such as sesame oil, olive oil, and soybean oil; preservatives such as p-hydroxybenzoic acid esters; and flavorings such as strawberry flavor and peppermint.

Among pharmaceutical compositions suitable for parenteral administration, liquid preparations such as injections, drip infusions, and eye drops may be prepared preferably as sterile isotonic liquid preparations. For example, injectable preparations can be prepared using an aqueous medium consisting of a salt solution, a dextrose solution, or an aqueous medium mixture of a salt water and a dextrose solution.

The dose and the number of administrations of the pharmaceutical composition for use according to the present invention are not particularly limited and may be appropriately determined according to the type and expression level of the disease-causing protein with the abnormally expanded PolyQ stretch, the species, gender, age, body weight, and presence or absence of underlying diseases, administration form and the like so that the inhibitory effect of PolyQ aggregation of arginine can be sufficiently exhibited. For example, in the case of oral administration or intravenous administration, the daily dose of the active ingredient for adults is preferably 0.1 to 10 g/kg (body weight) as arginine, more preferably 0.25 to 5.0 g/kg (body weight) as arginine, even more preferably 0.5 to 5.0 g/kg (body weight) as arginine, and still more preferably 1.0 to 5.0 g/kg (body weight) as arginine. Such a dose may be administered once or in several divided doses. For example, an orally administered preparation containing arginine as an active ingredient can be divided into three times a day (in the morning, at noon and in the evening) so that the total dose of arginine to be administered per day is 0.25 to 5.0 g/kg (body weight).

The PolyQ diseases gradually develop and progress slowly. By administering the pharmaceutical composition for use according to the present invention, aggregation of PolyQ is suppressed, so that progression of various symptoms derived from the PolyQ diseases is delayed. In other words, in order to suppress the progression and onset of symptoms, it is necessary to continuously take an aggregation inhibitor of PolyQ. The pharmaceutical composition for use according to the present invention containing arginine as an active ingredient which is safe even when administered stably for a long period of time is preferable as a therapeutic agent for patients having a PolyQ disease according to the present invention selected from the group consisting of inherited spinocerebellar ataxias, dentatorubral palliodoluysian atrophy, and spinal bulbar muscular atrophy. The pharmaceutical composition for use according to the present invention is particularly effective for treatment of patients with a low severity PolyQ disease according to the present invention, i.e. early PolyQ disease patients with less advanced neurodegeneration due to PolyQ aggregation. The pharmaceutical composition is also particularly effective for prevention before the onset of PolyQ disease according to the present invention on patients who have abnormal expansion of CAG repeats in the PolyQ disease-causing gene but have not yet developed symptoms (PolyQ disease pre-onset patients). By continuously taking the pharmaceutical composition for use according to the present invention, the onset of symptoms and the progression of symptoms can be effectively suppressed in patients with relatively mild PolyQ disease according to the present invention and patients in the pre-onset of PolyQ disease according to the present invention and their QOL can be improved.

The severity of spinocerebellar ataxia including PolyQ disease can be evaluated by, for example, modified Rankin Scale (mRS). The severity of mRS is evaluated as follows. Conditions without any symptoms are evaluated as 0. Conditions having some symptoms but no obvious obstacles, more specifically, conditions having subjective symptoms and objective signs without hindrance in daily life are evaluated as 1. Mildly impaired conditions, more specifically, conditions in which, although there are restrictions on the work and activities that have been done before onset, daily life can be done independently are evaluated as 2. Moderately disabled conditions, more specifically, conditions in which some kind of assistance is required, but assistance for walking, meals, and excretion is not required are evaluated as 3. Moderately to severely disabled conditions, more specifically, conditions in which constant care is not required, but walking and physical demands require assistance are evaluated as 4. Severely disabled conditions, more specifically, conditions requiring continuous nursing care and continuous monitoring, such as being bedridden are evaluated as 5. Death are evaluated as 6. The pharmaceutical composition for use according to the present invention is preferably continuously administered orally to a patient with spinocerebellar ataxia with mRS of 2 or less, preferably mRS of 0 or 1.

The severity of spinocerebellar ataxia can also be evaluated by, for example, The Scale for the Assessment and Rating of Ataxia (SARA) (Schmitz-Hubsch, et al., Neurology, 2006, vol. 66(11), p.1717-1720). The pharmaceutical composition for use according to the present invention is preferably continuously administered orally to patients with spinocerebellar ataxia with SARA of 20 or less, and more preferably 15 or less.

### Examples

Next, the present invention will be described in more detail by the following examples.

### <Protein Aggregation Turbidity Assay>

In the subsequent experiments, the protein aggregation turbidity assay was performed by the method described in Non-Patent Document 6. Specifically, it was performed as follows.

A solution in which abnormally expanded proteins were dissolved in physiological saline phosphate (PBS) was used as a measurement sample. The solution was incubated at 37 °C, and turbidity at 405 nm was measured every 24 hours using a microplate reader "Spectramax Plus plate reader" (Molecular Devices Inc.).

### <Statistical Analyses>

Turbidity, FRET, and mouse brain arginine concentration data were analyzed by one-way analysis of variance (ANOVA) followed by Tukey's multiple comparison test to assess for significant differences between individual groups. FCS measurements, tail movement in the tail-flick test, the relative amount of oligomers in the C. elegans model, and the percentage of cells with polyglutamine inclusions in mouse brains with or without arginine treatment, were analyzed by the Student t-test. Daily water or 6% arginine intake, rotarod, open-field activity, rearing, and balance beam data were analyzed by two-way ANOVA followed by Tukey's multiple comparison test. Survival data was analyzed using the log-rank test. For analyses between two groups, a Student's t-test was used. For all analyses, p < 0.05 was considered to indicate a statistically significant difference among groups. The software "GraphPad Prism" (GraphPad Software Inc.) was used for statistical analysis.

### <Animal experimentation>

All animal experiments were performed in accordance with the guidelines of the Animal Ethics Committee of the National Institute of Neuroscience in the National Center of Neurology and Psychiatry, Japan. Mice were housed on a 12-hour light/dark cycle with food and water provided ad libitum. Each mouse was weaned at either 3 or 5 weeks of age and maintained in cages with water bottles containing either water, 2% or 6% L-arginine.

### [Example 1]

The present inventors established an in vitro screening assay system capable of conveniently evaluating the aggregation of abnormally expanded PolyQ protein by the turbidity method (U.S. Pat. No. 6,632,616). Furthermore, the present inventors screened PolyQ aggregation inhibiting compounds using this abnormally expanded PolyQ protein aggregation assay system, and as a result, it was found that arginine has PolyQ aggregation inhibitory activity.

Therefore, the PolyQ aggregation inhibitory activity of arginine was examined in more detail. As an abnormally expanded PolyQ protein, a Thioredoxin-Q62 (Thio-Q62) fusion protein consisting of Thioredoxin and a PolyQ stretch with 62 glutamines was used.

### <Thio-Q62 protein preparation>

The preparation of Thio-Q62 protein was performed by the method described in Non-Patent Document 7. Specifically, it was performed as follows.

First, the Thio-Q62 protein was expressed in Escherichia coli DH5α and purified using a nickel column "nickel-chelating ProBond Resin columns" (Thermo Fisher Scientific). The purified Thio-Q62 protein was further purified by anion exchange chromatography using an HPLC apparatus "Äkta Explore HPLC" (GE Healthcare Life Sciences) equipped with a column "HiTrap Q Sepharose column" (GE Healthcare Life Sciences). The final concentration of the purified Thio-Q62 protein was determined by the Lowry method using a kit "DC protein assay kit" (Bio-Rad Laboratories). This purified Thio-Q62 protein was used for the following experiments.

### < Effect of Arginine on Protein Aggregation Rate >

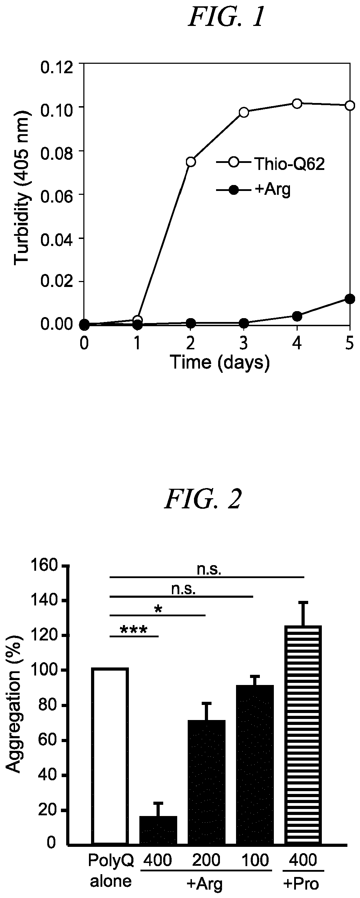

8.2 µM Thio-Q62 protein was incubated at 37 °C with 600 mM arginine in PBS and was subjected to turbidity measurement every 24 hours. As a control, Thio-Q62 protein alone was incubated in the same manner, and the turbidity was measured every 24 hours. The measurement results are shown in Fig. 1. In the figure, white circles represent turbidity of a solution containing Thio-Q62 protein alone, and closed circles represent turbidity of a solution containing Thio-Q62 protein and arginine. As the results of solutions with Thio-Q62 protein alone show, the turbidity of thio-Q62 solution increases in a time-dependent manner with a short lag phase followed by a rapid elevation during the aggregation phase and eventually reaches a plateau. In contrast, co-incubation of thio-Q62 with arginine at a neutral pH elongated the lag phase and decreased the speed of aggregation.

### < Dose-dependency of the inhibitory effect of arginine on polyQ aggregation >

10 µM thio-Q62 protein was co-incubated at 37 °C with 100, 200 or 400 mM arginine in PBS and its turbidity was measured after 5 days. As controls, a solution containing Thio-Q62 protein alone, and a solution containing Thio-Q62 protein and 400 mM proline were incubated in the same manner, respectively, and turbidity was measured after 5 days. Proline is a substance confirmed to have no PolyQ aggregation-inhibitory effect in the abnormally expanded PolyQ protein aggregation assay. The relative turbidity (%) of each solution was measured. The turbidity of the solution containing Thio-Q62 protein alone was set to 100%. The measurement results are shown in Fig. 2. In the figure, values represent means ± SEM (n = 3). "*" represents p < 0.05, "***" represents p < 0.001, "n.s." represents not significant. Proline did not exert any anti-aggregation effect, whereas it was found that, in the solution to which arginine was added, the relative turbidity was lower as arginine concentration was higher, and PolyQ aggregation inhibitory effect of arginine increased in a concentration-dependent manner.

### <Effect of arginine on seed-induced polyQ aggregation>

In amyloidogenic proteins such as polyQ, preformed protein aggregates are known to trigger aggregation of soluble protein monomers with a shortened lag phase, which is called seeding effects (Non-Patent Documents 7 and 8). Therefore, the effect of arginine on seeding effects was examined.

### (Seed preparation)

Thio-Q62 protein aggregates used as seeds were prepared as follows. First, purified Thio-Q62 solution (10µM) was snap frozen in liquid nitrogen, and stored at -80 °C until use. The frozen Thio-Q62 was thawed rapidly at 37 °C and centrifuged at 15,000 rpm for 5 minutes at room temperature. The supernatant was incubated at 37 °C for 6 days until the turbidity reached a plateau and then sonicated on ice for six pulses of 30 seconds each followed by a 60 seconds interval. As a result, Thio-Q62 protein aggregates to be seed was formed. The resulting sonicated solution was used as a seed solution for subsequent experiments.

### (Seed-induced polyQ aggregation reaction)

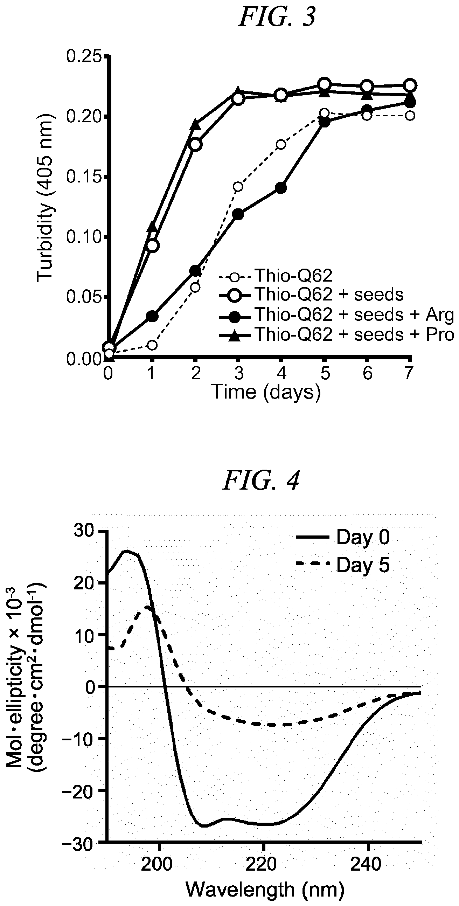

Thio-Q62 protein aggregate seeds, arginine, and proline were added to 10 µM soluble Thio-Q62 protein solution as shown in FIG. 3, incubated at 37 °C for 7 days, and was subjected to turbidity measurement every 24 hours. The addition of the seed was performed by adding 20 µL per 200 µL of the Thio-Q62 protein solution to the seed solution previously formed as described above. Arginine and proline were both added to a final concentration of 400 mM per 200 µL of Thio-Q62 protein solution. Changes with time of turbidity of each solution are shown in FIG. 3. As a result, compared with the solution containing soluble Thio-Q62 protein alone ("Thio-Q62" in the figure), in the solution containing Thio-Q62 protein with added seed alone ("Thio-Q62+seeds" in the figure), the lag phase was disappeared and an immediate increase in turbidity from day 1 was observed, demonstrating a seeding effect. Those results indicate that a seed effect has been observed in the solution containing Thio-Q62 protein with added seed alone. In the solution containing Thio-Q62 protein with added seed and arginine("Thio-Q62+seed+Arg" in the figure), the seed-induced increase in the turbidity was markedly decreased, while the effects as observed with arginine was not obtained in the solution containing Thio-Q62 protein with added seed and proline("Thio-Q62+seed+Pro" in the figure). These results indicate that arginine alleviated the seed-induced polyQ aggregation and hence suggest the potential of arginine to decelerate further aggregation of polyQ protein after the initiation of the aggregation process.

### [Example 2]

Aggregation-prone proteins undergo multiple processes before the formation of insoluble aggregates such as misfolding and beta-sheet conformation transition of the protein monomer and assembly of the monomers into oligomers. Recent studies have indicated that aggregation-prone proteins including proteins with expanded polyQ stretch exert cellular toxicity before the formation of insoluble aggregates, when proteins undergo conformation transition to β-sheet rich structure and assemble into soluble oligomers (Non-Patent Document 9). That is, in the case of aggregation-prone proteins, the misfolded and oligomerized soluble protein species rather than the insoluble aggregates exhibit toxicity in the cells and brains.

Since arginine elongated the lag phase of thio-Q62 solution (Example 1) and hence was suggested to affect these initial processes before insoluble aggregate formation, it was investigated whether arginine alters the formation of these soluble but toxic protein species. Specifically, the effect of arginine on the β-sheet conformational transition of the polyQ protein and oligomer formation was investigated.

### <CD (Circular Dichroism) analysis>

The thio-Q62 protein undergoes a conformational transition from an α-helix- to a β-sheet-rich structure in the monomeric state and that the β-sheet monomer of the thio-Q62 protein exerts cellular toxicity (Non-Patent Document 7). This conformational transition of thio-Q62 was confirmed by circular dichroism analysis. Specifically, 8.2 µM Thio-Q62 protein was incubated in PBS (pH 7.5) at 37 °C for five days. CD spectra of Thio-Q62 protein in PBS (pH 7.5) before and after incubation were measured respectively, using a spectropolarimeter Model J-820 (Jasco) as described in Non-Patent Document 7. The measurement results are shown in Fig. 4. The spectra of Fig. 4. are presented as an average of 10 scans recorded at a speed of 50 nm/min and a resolution of 1 nm. The data are expressed as molar residue ellipticity (θ). CD spectrum of the Thio-Q62 protein before incubation had a large positive maximal value around 196 nm, and had negative maximal values around 207 nm and around 222 nm, respectively ("Day 0" in the figure). From the CD spectrum, it was confirmed that the Thio-Q62 protein before incubation formed an α-helix structure. The CD spectrum of the Thio-Q62 protein after incubation had a positive maximal value around 197 nm and a negative maximal value around 216 nm. From the CD spectrum, it was confirmed that the Thio-Q62 protein after incubation formed a β-sheet structure.

### <Native PAGE and Turbidity measurement

The effect of arginine on the β-sheet conformational transition of the polyQ protein was investigated by native non-denaturing polyacrylamide gel electrophoresis (PAGE). Specifically, 8.2 µM Thio-Q62 protein was incubated with or without 600mM arginine in PBS (pH 7.5) at 37 °C for five days. Sampling for native PAGE and turbidity measurement were performed before incubation and every 24 hours after incubation. As a control, Thio-Q62 protein alone was incubated in the same manner, and sampling and turbidity measurement were performed. The sample solutions were subjected to 10% (w/v) PAGE without SDS under non-denaturing conditions and separated proteins in the solutions. The separated proteins were stained with Coomassie brilliant blue (CBB).

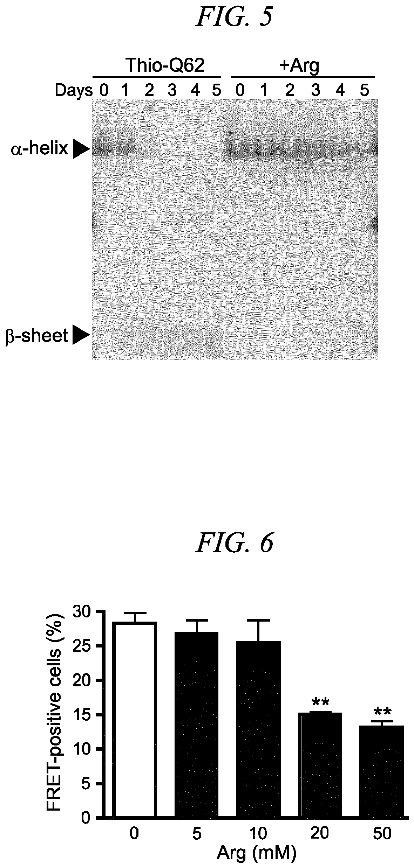

The results of the CBB staining are shown in Fig. 5. As a result, in the absence of arginine, the band corresponding to the β-sheet-rich monomer gradually appeared upon incubation and the band corresponding to the α-helix-rich monomer gradually disappeared. On the other hand, in the presence of arginine, the band corresponding to the β-sheet-rich monomer did not appear even when incubated for a sufficient period of time, suggesting that arginine inhibited the toxic β-sheet conformational transition of Thio-Q62.

Incubation of Thio-Q62 without arginine caused the turbidity to rise sharply on day 2 from the start of the incubation (FIG. 1). In contrast, even though the band of β-sheet-rich monomer was confirmed on day 1 from the start of incubation, there was no increase in turbidity at this time.

### <FRET Analysis>

The effect of arginine on polyQ oligomer formation in cultured cells was investigated using fluorescence resonance energy transfer (FRET). PolyQ protein fused with the fluorescent protein CFP and PolyQ protein fused with the fluorescent protein YFP assemble together into soluble oligomers in cultured cells and result in a positive FRET signal arosen from the direct interaction between polyQ stretches. In other words, the formation of soluble oligomers can be identified in living cells by a positive FRET signal.

Cell culture and FRET analyses were performed by the method described in Non-Patent Document 10. Specifically, first, COS-7 cells were prepared which co-express a protein having a PolyQ repeat consisting of 56 glutamines fused with CFP (Q56-CFP) and a protein having a PolyQ repeat consisting of 56 glutamines fused with YFP (Q56-YFP). COS-7 cells expressed those fluorescent Protein-Fused PolyQ Proteins were prepared by transfecting the expression vector for each protein using lipofectamine 2000 (Life Technologies). Forty-eight hours after transfection, cells were subjected to FRET analyses.

Cells expressing Q56-CFP and Q56-YFP were incubated in medium containing 0-50 mM arginine and the average FRET signal intensity was determined after 48 hours. A numerical cutoff was calculated on the basis of the mean and 95% confidence interval for the FRET signal intensities of COS-7 cells co-expressing Q56-CFP and Q56-YFP. Only cells showing FRET signal intensities above the cutoff value were considered to be FRET-positive cells. The mean FRET signal intensity of 150 cells was analyzed. ANOVA and the Tukey post hoc test were used for the statistical analysis of the results. The results are shown in Fig. 6. Incubation with 20 or 50 mM arginine significantly decreased the percentage (%) of the FRET-positive cells. Incubation with lower concentrations of arginine did not affect the proportion of FRET-positive cells. These results demonstrate that arginine inhibits the interaction among the polyQ proteins assembling into oligomers in cultured cells in a dose-dependent manner.

### <FCS Analysis>

To confirm the results of FRET analyses, the effect of arginine on polyQ oligomer formation was quantitatively evaluated using Fluorescence Correlation Spectroscopy (FCS). For polyQ oligomer formation, the fluorescent protein GFP fused with a protein having a PolyQ repeat consisting of 45 glutamines (Q45-GFP) was used as the monomer. Two indices obtained from FCS measurement were used to assess the property of polyQ oligomers; counts per particle (CPP) and the diffusion time of the fast components (DT₁) (Non-Patent Document 11). CPP is the average fluorescent intensity of the fluorescent particles measured and hence directly reflects the number of Q45-GFP monomers per oligomer. DT₁ reflects the size of the Q45-GFP oligomers.

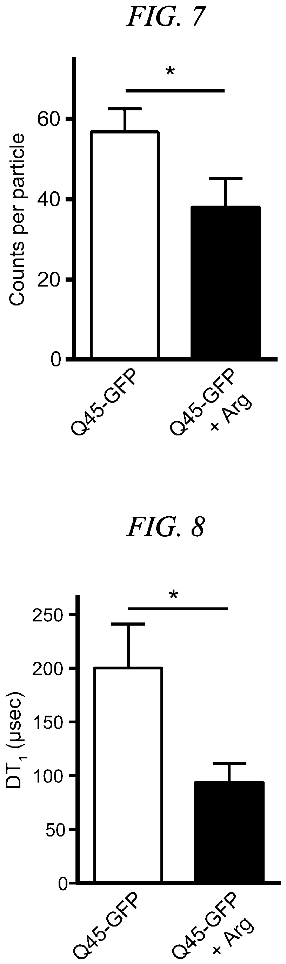

FCS analysis was performed by the method described in Non-Patent Document 11. Specifically, first, COS-7 cells were transfected with expression vectors for the Q45-GFP protein, incubated in media with or without 25 mM arginine and lysed at 48 hours after transfection. The cell lysates were gently sonicated and centrifuged to remove insoluble Q45-GFP inclusion bodies. The resulting supernatants containing no visible inclusion bodies were subjected to FCS measurements. From the measurements, the diffusion time, which corresponds to the average time for diffusion of fluorescent particles across the detection area and CPP, which directly reflects the number of fluorescent molecules per particle, were calculated. The results of CPP and DT₁ are shown in Fig. 7 and 8, respectively. CPP and DT₁ in cells treated with arginine were decreased compared to untreated cells. These results also indicate that arginine inhibits oligomer formation of the polyQ protein within cells.

To summarize the results of FRET analysis and FCS analysis, these results indicate that arginine inhibits the toxic β-sheet conformational transition and oligomer formation of the polyQ protein, and hence suggest that arginine has an ability to interfere with the initial processes of polyQ protein aggregation in which misfolded polyQ proteins exert neuronal toxicity.

### [Example 3]

The therapeutic effects of arginine in animal models of polyQ diseases were investigated. Two well-established models in invertebrates, a Drosophila melanogaster model and a Caenorhabditis elegans model were used.

### <The effects of arginine on the Drosophila model of the polyQ diseases>

The spinocerebellar ataxia type 3 (SCA3/MJD) model flies were used, which express the truncated form of the MJD protein with an abnormally expanded polyQ repeat consisting of 78 glutamines (MJDtr-Q78) in the compound eye (Non-Patent Document 12). The severe compound eye degeneration induced by MJDtrQ78 can be easily detected under the light microscope.

Fly culture and crosses were performed under standard conditions at 25 °C. The MJDtr-Q78(S) transgenic fly line bearing the UAS-MJDtr-Q78 transgene for expression of MJDtr-Q78 described in Non-Patent Document 12 was used. The fly line bearing the gmr-GAL4 transgene was obtained from the Drosophila Genetic Resource Center at Kyoto Institute of Technology, Japan. Flies were fed arginine by culturing them in "Instant Drosophila Medium" (Carolina Biological Supply Company) containing 1-100 mM arginine. For evaluation of compound eye degeneration, light microscopic images of the compound eye were taken using a stereoscopic microscope model "SZX9" (Olympus). For detection of the MJDtr-Q78 protein in eye imaginal discs of third instar larvae, immunostaining with an anti-hemagglutinin antibody (clone 3F10, Roche) was performed according to the method described in Non-Patent Document 13. Fluorescence microscopic images were taken using a confocal laser scanning microscope model "LSM510" (Carl Zeiss).

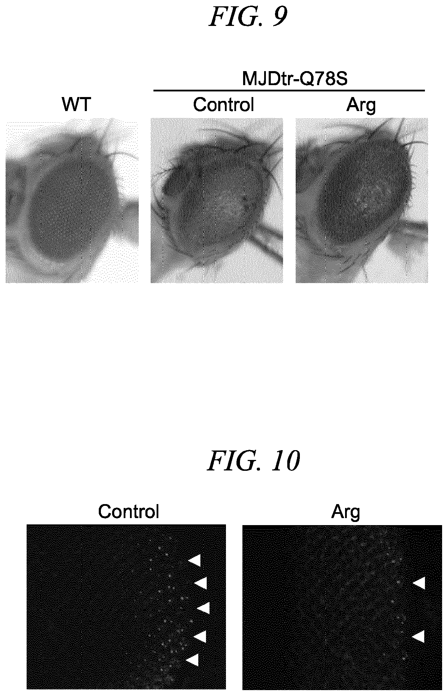

Representative light microscopic images of the external compound eyes of wild-type and MJDtr-Q78(S) flies are shown in Fig. 9. In the figure, "WT" represents a compound eye of a wild-type fly and "Control" represents a compound eye of a MJDtr-Q78(S) fly without arginine administration, and "Arg" represents a compound eye of a MJDtr-Q78(S) fly with 100 mM arginine administration. The compound eye degeneration observed in MJDtr-Q78(S) flies were remarkably ameliorated by administrating food containing either 25, 50 or 100 mM arginine.

Immunostaining images with anti-hemagglutinin antibody of the eye discs of larvae of MJDtr-Q 78 (S) flies are shown in Fig. 10. These immunostaining images were taken using a confocal microscope. The portion stained with the anti-hemagglutinin antibody indicated by white arrowheads in the figure shows inclusion bodies formed in eye discs. In the figure, "Control" represents a compound eye of an MJDtr-Q78(S) fly without arginine administration, and "Arg" represents a compound eye of an MJDtr-Q78(S) fly with 100 mM arginine administration. The number of the inclusion bodies in the eye discs of MJDtr-Q78(S) fly larvae were decreased by administrating food containing arginine. These results suggested that arginine administration suppresses the formation of inclusion bodies containing PolyQ.

### <The effect of arginine on the C. elegans model of the polyQ diseases>

As a C. elegans model of the polyQ diseases, a Q40-GFP expressing nematode line (Non-Patent Document 14), which is a C. elegans line expressing GFP having a polyQ repeat consisting of 40 glutamines (Q40-GFP) was used. Worms of this line exhibit motor dysfunction as assessed by the tail-flick test and shorter survival.

Nematodes were grown at 20 °C on nematode growth medium (NGM) plates seeded with Escherichia coli OP50. For the generation of the Q40-GFP expression nematode line, plasmids encoding Q40-GFP were injected into wild type strain N2.

Tail-flick tests were performed on Q40-GFP expressing nematode line worms to examine the effect of arginine on motor dysfunction. Tail-flick tests were performed at 20 °C on nematodes synchronized to 6 days old. First, each nematode was transferred to a separate plate. One drop of S-basal solution was dropped on one nematode, and after waiting for 5 minutes for them to recover, the number of movements of the tail of the worm was measured for 1 minute. The number of movements of the tail was defined as the number of times to change the bending direction of the mid-body of a nematode.

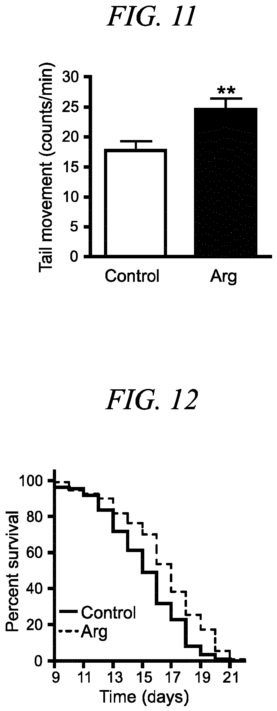

The measurement results of tail movements per minute (count/min) obtained from the tail flick tests are shown in Fig. 11. In the figure, "Control" represents the results of the Q40-GFP expressing nematode worm line without arginine administration, and "Arg" represents the results of the Q40-GFP expressing nematode worm line with 10 mM arginine administration. Values represent means ± SEM (n = 48) and "**" represents p < 0.01. The tail movements of worms of the Q40-GFP expressing nematode line were significantly improved by 10 mM arginine administration.

To analyze the effect of arginine on the decreased survival of the Q40-GFP expressing nematode worm line, lifespan analysis was performed. For lifespan analysis, nematodes were cultured on OP50 bacteria, and were examined daily for signs of life. Worms that did not move after vigorous stimulation were considered dead. The time courses of the survival rates of worms of the Q40-GFP expressing nematode line are shown in Fig. 12. In the figure, "Control" represents the results of the Q40-GFP expressng nematode worm line without arginine administration, and "Arg" represents the results of the Q40-GFP expressing nematode worm line with 10 mM arginine administration (the log-rank test: P<0.01). The survival rate of worms of the Q40-GFP expressing nematode line was significantly improved by 10 mM arginine administration.

Semi-denaturing detergent agarose gel electrophoresis (SDD-AGE) was performed on the Q40-GFP expressing nematode worm line to examine the effect of arginine on oligomer formation of polyQ protein. SDD-AGE was performed as follows. First, nematodes were lysed in lysis buffer (0.5% SDS, 0.5% NP-40, 50mM Tris-HCl, 150mM NaCl, 5mM EDTA, protease inhibitor cocktail), the resulting lysate was run on a 1 % agarose gel with 0.01% SDS in a running buffer of Tris-Glycine containing 0.01% SDS, and then transferred overnight onto a nitrocellulose membrane. For subsequent western blot analysis, the membrane was incubated with the anti-GFP antibody (MBL Life sciences) at 10,000 dilution as the primary antibody at 4 °C overnight, and then with the HRP-conjugated anti-rabbit IgG antibody (MBL Life sciences) at 20,000 dilution as the secondary antibody. Immunoblots were imaged using the Lumino image analyzer system "ImageQuant LAS 4000" (GE Healthcare Life Sciences) and analyzed using "ImageQuantTL" (GE Healthcare Life Sciences).

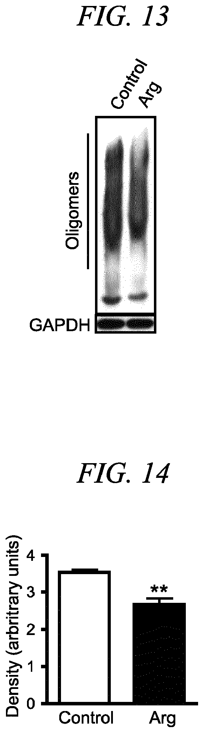

The SDD-AGE image obtained by the immunoblotting of nematode lysates is shown in Fig. 13, and the measurement results of the smear intensity in the SDD-AGE image are shown in Fig. 14, respectively. In the figures, "Control" represents the results of the Q40-GFP expressing nematode worm line without arginine administration, and "Arg" represents the results of the Q40-GFP expressing nematode worm line with 10 mM arginine administration. In Fig. 14, values represent means ± SEM (n = 5) and "**" represents p < 0.01. While the polyQ disease worms (Q40-GFP expressing nematode line) without arginine exhibited smearing of Q40-GFP oligomers on the membrane, the polyQ disease worms with arginine showed a much lighter and less broad smear (Fig. 13), the smear intensity measured by image analysis also became smaller. These results suggested that oral administration of arginine could suppress polyQ protein oligomerization in the PolyQ disease nematodes.

From the results of the administration of arginine to the PolyQ disease model flies and nematodes, it is clear that arginine exerts therapeutic effects in vivo in invertebrate models of the polyQ diseases via the inhibition of polyQ protein oligomerization and aggregation.

### [Example 4]

The Scal^{154Q/2Q} knock-in mouse (hereinafter, simply referred to as "SCA1 mouse") (Non-Patent Document 15), which is one of mouse models of spinocerebellar ataxia type 1 was used to confirm the therapeutic effect of arginine in mammals. SCA1 mice express the abnormally expanded polyQ protein (the mutant ataxin 1 protein with polyQ repeats consisting of 154 glutamines) at endogenous levels and with accurate temporal and spatial patterns, and replicates numerous aspects of the human disease including progressive motor deficit. The SCA1 mice used in this experiment were a kind gift from Dr. Kei Watase (Tokyo Medical and Dental University, Japan).

### < BBB permeability of arginine >

To confirm the BBB permeability of arginine, arginine was orally administered to wild type mice and SCA1 mice, and the amounts of arginine in the brain and serum were measured.

Specifically, first, WT or SCA1 mice were orally administered once an hour with 200 µL of water, 5% or 15% L-arginine aqueous solution using an oral gavage needle for nine hours. The total amount of L-arginine administered during this period was equal to the total amount of the daily intake of L-arginine by the mice administered with 2% or 6% L-arginine aqueous solution ad libitum respectively (data not shown). After one hour from the final administration, the mice were anesthetized with pentobarbital and euthanized by exsanguination from the abdominal aorta. The blood was incubated at room temperature for 1 hour and centrifuged to obtain the serum. The brains were excised after perfusion with 10 mL saline and dissected into cerebral hemispheres. The serum and the cerebrum were flash-frozen and stored at -80 °C until use, and were subjected to L-arginine detection. L-arginine detection was performed using a two-dimensional HPLC system, according to the method described in Non-Patent Document 16.

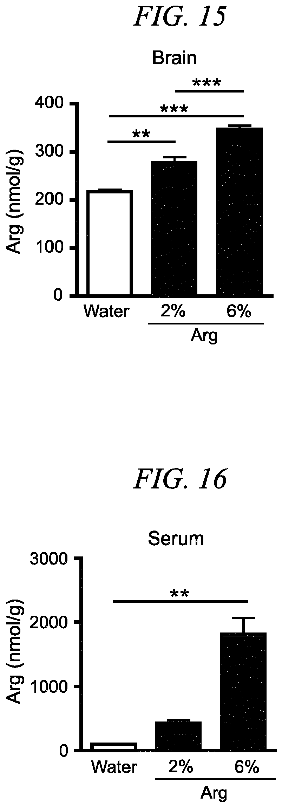

The measurement results of the amount of L-arginine in the cerebrum of SCA1 mice are shown in Fig. 15, and the measurement results of the amount of L-arginine in the serum of SCA1 mice are shown in Fig. 16, respectively. In the figures, "Water" represents the results of mice administrated water alone, "2 %" represents the results of mice administrated 5 % L-arginine aqueous solution, and "6 %" represents the results of mice administrated 15 % L-arginine aqueous solution. In both figures, values represent means ± SEM (n = 3), "**" represents p < 0.01, and "***" represents p < 0.001, respectively. The amount of L-arginine in serum and cerebrum in mice was increased depending on the amount of orally administrated L-arginine (Fig. 15 and 16). This confirmed that oral administration of arginine to mice led to a dose-dependent increase in the concentrations of arginine in sera and brains.

### < Effect of arginine on the function of SCA1 mice >

To examine the therapeutic effect of arginine, SCA1 mice were continuously administered with 6% by weight of arginine in drinking water (arginine treated) from 3 weeks of age and subjected to rotarod test, balanced beam analysis and PolyQ inclusion body analysis in the brain. As a control, water without arginine was given to SCA1 mice as drinking water (arginine untreated).

### (1) Rotarod test

Rotarod tests have been previously utilized for the assessment of motor function in SCA1 mice (Non-Patent Document 15). SCA1 mice showed a clear deficit at 4 weeks of age, which steadily progressed until 36 weeks of age when the analysis was ended

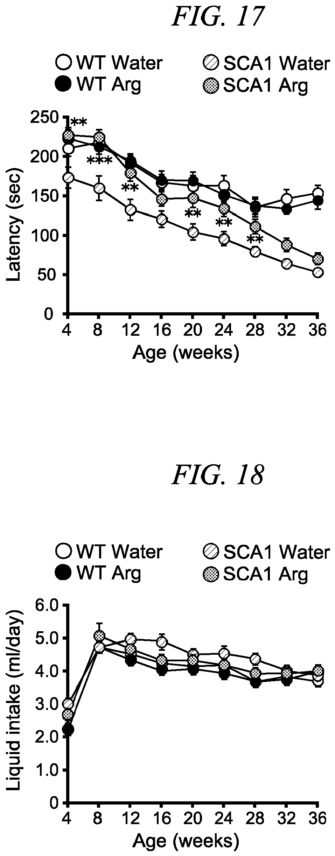

For rotarod analysis, mice were tested on an accelerating rotarod apparatus (Ugo Basile) and performed according to the method described in Non-Patent Document 17. The time courses of the amount of walking time until wild type mice and SCA1 mice fell from the rotarod (Latency) are shown in Fig.17. In the figure, "WT Water" represents the results of wild type mice administered water without arginine, " WT Arg" represents the results of wild type mice administered water with arginine, and "SCA1 Water" represents the results of SCA1 mice administered water without arginine, "SCA1 Arg" represents the results of SCA1 mice administered water with arginine. In the figure, values represent means ± SEM (n = 16-18), "**" represents p < 0.01, and "***" represents p < 0.001, respectively.

As shown in Fig.17, the motor function of SCA1 mice treated with 6% by weight arginine was significantly better than the untreated mice from 4 to 12 weeks of age, and was comparable to the WT control mice, while arginine administration to WT mice did not exert any obvious effects. The therapeutic effect of arginine remained significant until 28 weeks of age. It was confirmed that all mouse groups had a comparable amount of liquid intake (Fig. 18), which suggests that addition of arginine in the drinking water did not affect the *ad libitum* drinking activity of WT or SCA1 mice.

### (2) Balance beam analysis

SCA1 mice were also examined using the balance beam test to assess the motor coordination. For balance beam analysis, mice were assessed on their time to cross a beam with 1 m-long and 15 mm-diameter. The beam was placed approximately 50 cm above the ground with a bright light at the starting end and a dark box at the far end. Mice were initially trained for 3 consecutive days, 2 trials each, so that they learn to cross the beam without falling or turning around. On testing days, the time it took the mice to cross the beam was recorded. If the mice took longer than 60 seconds to cross, the score was counted as 60 seconds. Two trials were performed for each mouse, and a faster time was used for analysis.

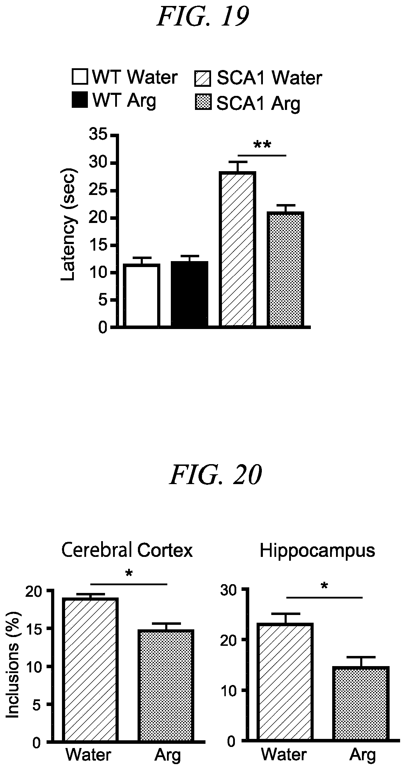

The time courses of the amount of time to cross the beam (Latency) of wild type mice and SCA1 mice at 28-weeks-old are shown in Fig.19. In the figure, "WT Water", "WT Arg", "SCA1 Water", and "SCA1 Arg" represent the same as in Fig.17. In the figure, values represent means ± SEM (n = 16-18), "**" represents p < 0.01, and "***" represents p < 0.001, respectively. In SCA1 mice, deficit on the balance beam was observed at a later age compared with the rotarod deficit. At 28 weeks of age, untreated SCA1 mice required more than twice the amount of time to cross the beam compared with the untreated WT mice. Arginine-treated SCA1 mice were able to cross the balance beam in significantly less time compared with untreated SCA1 mice. From these results, it was confirmed that oral administration of arginine can improve the motor dysfunction of PolyQ disease animals.

### (3) Effect on inclusion body formation

The effect of arginine on the inclusion body formation in the SCA1 mice brain was analyzed. It was reported that SCA1 mice develop polyQ inclusion bodies (PolyQ protein aggregates) in their neurons of the cerebral cortex and hippocampus from around 4 weeks of age (Non-Patent Document 15).

PolyQ inclusion bodies in brain sections were detected using immunohistochemical analysis. First, ten-micrometer-thick frozen sections of 12 week-old SCA1 mouse brains were prepared according to the method described in Non-Patent Document 17. The sections were blocked in PBS containing 5% goat serum and 0.1% Triton X-100 for 1 hour at room temperature, then incubated with a rabbit anti-ubiquitin antibody (clone FK2, 1:500) at 4 °C overnight, followed by an Alexa Fluor 488-conjugated goat anti-rabbit IgG antibody (1:1,000, Life Technologies) for 1 hour at room temperature. The sections were mounted with antifade reagent "Slowfade Gold antifade reagent with DAPI" (Life Technologies) and examined using a confocal laser-scanning microscope "FV 1000" (Olympus).

By confocal laser scanning microscopy, inclusion bodies stained with the anti-ubiquitin antibody can be visualized. Therefore, the sections were observed using a confocal laser scanning microscope, cells included in the field of view were counted, and the percentage (%) of the cells in which the inclusion bodies were formed to the total number of cells was measured. In each mouse, the cell count was averaged for 3 fields of view, in which over 130 or 70 cells were counted in the cortex or hippocampus, respectively. The measurement results are shown in Fig.20. In the figure, "Water" represents the results of SCA1 mice administered water without arginine, "Arg" represents the results of SCA1 mice administered water with arginine. In the figure, values represent means ± SEM (n = 3 or 4), and "*" represents p < 0.05. Immunohistochemical analyses of the brain sections of 12-week-old SCA1 mice confirmed the formation of PolyQ inclusion bodies in the cerebral cortex and the hippocampus. In arginine-treated mice, the percentage of cells with inclusion body formation in both regions was significantly decreased.

That is, oral administration of arginine to SCA1 mice improves motor dysfunction and suppresses the formation of PolyQ inclusion bodies in the brain. From these results, it is clear that arginine has a therapeutic effect on the PolyQ diseases.

### [Example 5]

### < Effect of arginine on SCA1 mice after disease onset of motor dysfunction >

An important factor of consideration in developing treatments for neurodegenerative diseases including polyQ diseases is whether the drug exerts its therapeutic effect at the symptomatic phase when the pathological process has already progressed. To date, therapeutic candidates have been administered to polyQ disease mice prophylactically at the pre-symptomatic phase in most preclinical studies. To test whether arginine administration is also effective at the symptomatic phase, arginine was administered to SCA1 mice from 5 weeks of age, when these mice already show neurological deficits.

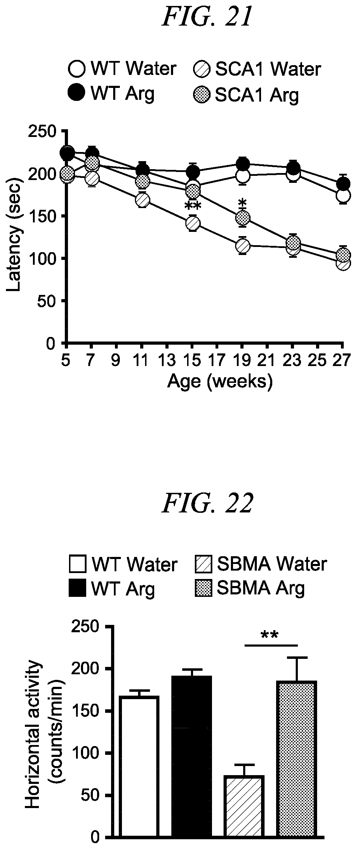

Specifically, SCA1 mice were continuously administered with 6% by weight of arginine in drinking water from 5 weeks of age, and subjected to the rotarod test to examine the therapeutic effect of arginine. The rotarod test was carried out in the same manner as in Example 4. The time courses of the amount of walking time until wild type mice and SCA1 mice fell from the rotarod (Latency) are shown in Fig.21. In the figure, "WT Water", "WT Arg", "SCA1 Water", and "SCA1 Arg" represent the same as in Fig.17. In the figure, values represent means ± SEM (n = 23-25), "*" represents p < 0.05.

As shown inn Fig.21, motor dysfunction was observed in 5-week-old SCA1 mice before arginine treatment, demonstrating significantly shorter time to walk without falling from the rotarod compared to wild type mice. Thereafter, arginine-treated SCA1 mice had a longer time to walk without falling from the rotarod than the untreated SCA1 mice from 7 weeks of age to 19 weeks of age, demonstrating that motor dysfunction was alleviated. Indeed, the differences in motor function between arginine-treated SCA1 mice and untreated SCA1 mice reached statistical significance at 15 weeks and 19 weeks of age (Fig.21). These results demonstrate that arginine exerts therapeutic effects on the neurological phenotypes of the polyQ diseases even when administration is started after the onset of motor symptoms, indicating that arginine is very promising not only as a preventive agent but also as a therapeutic agent against the PolyQ diseases.

### <Effect of arginine on the function of SBMA mice>

All PolyQ diseases arise from abnormal expansion of PolyQ chain in each disease-causing protein and arginine has an inhibitory effect on the abnormally expanded PolyQ stretch itself. From these facts, whether arginine is also effective against another PolyQ disease model mouse was examined.

As another PolyQ disease mouse model, the SBMA mouse (Non-Patent Document 18) was used. The SBMA mouse model, which is transgenic for the full length protein of the human androgen receptor having an abnormal expanded PolyQ stretch consisting of 97 glutamines, shows progressive motor dysfunction. This motor dysfunction can be reversed with drug treatment (Non-Patent Document 19). The SBMA mouse used in this experiment was a kind gift from Dr. Gen Sobue (Nagoya University, Japan).

The motor dysfunction of the SBMA mouse can be monitored as spontaneous motor dysfunction, by measuring horizontal open-field activity and rearing behavior in the cage (Non-Patent Document 18). To examine the therapeutic effect of arginine, SBMA mice were continuously administered with 6% by weight of arginine in drinking water (arginine treated) from 3 weeks of age and, at 12 weeks of age, horizontal open-field activity and rearing behavior were analyzed. As a control, water without arginine was given to SBMA mice as drinking water (arginine untreated).

Horizontal open-field activity and rearing behavior were measured using a spontaneous locomotor activity monitor "Supermex" (Muromachi Kikai) according to the method described in Non-Patent Document 18. Specifically, a mouse was placed in a measuring cage in which infrared beams were projected inside and were allowed to move around freely. When the mouse moves inside the measuring cage, the infrared beam is obstructed and the infrared ray cannot be detected by the light receiving part (beam break). The number of beam breaks per minute was determined by measuring the number of beam breaks (counts/min). To detect movements in the horizontal direction, the number of beam breaks of the infrared beams projected in the vertical direction were counted. To detect movements in the vertical direction (the movement of mice standing on their hind legs), infrared beams were projected in the horizontal direction at a height in which the infrared beams were not obstructed when the mouse was on all four limbs, but were obstructed when the mouse stands on its hind legs, and the number of beam breaks was counted.

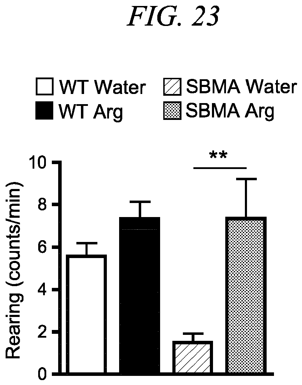

The observation results of horizontal activity and the observation results of rearing behavior are shown in Fig.22 and in Fig.23, respectively. In Fig.22-23, "WT Water" represents the results of wild type mice administered water without arginine", "WT Arg" represents the results of wild type mice administered water with arginine, and "SCA1 Water" represents the results of SCA1 mice administered water without arginine, and "SCA1 Arg" represents the results of SCA1 mice administered water with arginine. In the figure, values represent means ± SEM (n = 23-25), and "**" represents p < 0.01.

As a result, by treating SBMA mice with arginine, both horizontal activity and rearing behavior were improved to levels comparable to untreated wild type mice. Arginine treatment on wild type mice had limited effects. These results indicated that arginine is effective against many PolyQ disease mouse models, and furthermore, that arginine is effective as a therapeutic molecule against the PolyQ diseases in general.

## Claims

1. A pharmaceutical composition, comprising:
one selected from the group consisting of arginine, a physiologically acceptable salt thereof, and a solvate thereof, as an active ingredient,
for use in treatment or prevention of the PolyQ diseases and the PolyQ disease is one selected from the group consisting of inherited spinocerebellar ataxias, dentatorubral palliodoluysian atrophy, and spinal bulbar muscular atrophy.

2. The pharmaceutical composition for use according to Claim 1,
wherein the PolyQ disease is one selected from the group consisting of inherited spinocerebellar ataxia type 1, inherited spinocerebellar ataxia type 3, and spinal bulbar muscular atrophy.

## Patentansprüche

1. Pharmazeutische Zusammensetzung, umfassend
eines ausgewählt aus der Gruppe, bestehend aus Arginin, einem physiologisch akzeptablen Salz davon und einem Solvat davon, als ein Wirkstoff,
zur Verwendung bei der Behandlung oder Vorbeugung der PolyQ-Krankheiten und die PolyQ-Krankheit ist eine, ausgewählt aus der Gruppe, bestehend aus vererbten spinozerebellären Ataxien, dentatorubraler Palliodoluysian-Atrophie und spinaler bulbärer Muskelatrophie.

2. Pharmazeutische Zusammensetzung zur Verwendung nach Anspruch 1,
wobei die PolyQ Krankheit eine ist, ausgewählt aus der Gruppe, bestehend aus vererbter spinozerebellärer Ataxie Typ 1, vererbter spinozerebellärer Ataxie Typ 3 und spinaler bulbärer Muskelatrophie.

## Revendications

1. Composition pharmaceutique, comprenant :
un sélectionné dans le groupe constitué par l'arginine, un sel physiologiquement acceptable de celle-ci, et un solvate de celle-ci, en tant que principe actif,
pour son utilisation dans le traitement ou la prévention des maladies à PolyQ et la maladie à PolyQ est une sélectionnée dans le groupe constitué par les ataxies spinocérébelleuses héréditaires, l'atrophie dentato-rubro-pallido-luysienne, et l'atrophie musculaire spinale et bulbaire.

2. Composition pharmaceutique pour son utilisation selon la revendication 1,
dans laquelle la maladie à PolyQ est une sélectionnée dans le groupe constitué par l'ataxie spinocérébelleuse héréditaire de type 1, l'ataxie spinocérébelleuse héréditaire de type 3, et l'atrophie musculaire spinale et bulbaire.
